(19) Europäisches Patentamt / European Patent Office / Office européen des brevets

(11) **EP 2 948 768 B1**

(12) **EUROPÄISCHE PATENTSCHRIFT**

(45) Veröffentlichungstag und Bekanntmachung des Hinweises auf die Patenterteilung:
**15.05.2019  Patentblatt 2019/20**

(21) Anmeldenummer: **14705701.2**

(22) Anmeldetag: **22.01.2014**

(51) Int Cl.:
***G01N 33/14*** *(2006.01)*    ***G01N 21/41*** *(2006.01)*
***C12M 1/36*** *(2006.01)*

(86) Internationale Anmeldenummer:
**PCT/EP2014/000155**

(87) Internationale Veröffentlichungsnummer:
**WO 2014/114448 (31.07.2014 Gazette 2014/31)**

(54) **VERFAHREN ZUM STEUERN EINER GÄRUNG**

METHOD FOR CONTROLLING FERMENTATION

PROCÉDÉ DE CONTRÔLE D'UNE FERMENTATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: 22.01.2013   DE 102013100600
11.03.2013   DE 102013102368
11.12.2013   DE 102013020883

(43) Veröffentlichungstag der Anmeldung:
**02.12.2015   Patentblatt 2015/49**

(73) Patentinhaber: **Alber, Bernd**
**76227 Karlsruhe (DE)**

(72) Erfinder:
• **ALBER, Bernd**
**76133 Karlsruhe (DE)**
• **ROTHER, Herbert**
**69118 Heidelberg (DE)**
• **JACUBASCH, Andreas**
**68789 St. Leon-Rot (DE)**

(74) Vertreter: **Raible Deissler Lehmann Patentanwälte PartG mbB**
**Senefelderstrasse 26**
**70176 Stuttgart (DE)**

(56) Entgegenhaltungen:
EP-A2- 1 559 775    CN-U- 201 955 301
DE-T2- 3 787 515

• **George A Cojocaru ET AL: "A NEW REFRACTOMETRIC METHODOLOGY USED TO MONITOR FERMENTATIONS", , 31. Dezember 2012 (2012-12-31), XP55115712, Gefunden im Internet: URL:http://horticulturejournal.usamv.ro/pdf/vol12issue4/Art78.pdf [gefunden am 2014-04-29]**
• **JIMENEZ F ET AL: "Multi-purpose optoelectronic instrument for monitoring the alcoholic fermentation of wine", 2011 IEEE SENSORS PROCEEDINGS : LIMERICK, IRELAND, 28 - 31 OCTOBER 2011, IEEE, PISCATAWAY, NJ, 28. Oktober 2011 (2011-10-28), Seiten 390-393, XP032093392, DOI: 10.1109/ICSENS.2011.6127192 ISBN: 978-1-4244-9290-9**
• **Reginald Haydn Hopkins ET AL: "CCXCVII. THE KINETICS OF ALCOHOLIC FERMENTATION OF SUGARS BY BREWER'S YEAST. III. THE TEMPERATURE COEFFICIENTS OF THE RATES OF FERMENTATION OF GLUCOSE AND FRUCTOSE", , 28 September 1935 (1935-09-28), XP55160409, Retrieved from the Internet: URL:http://www.biochemj.org/bj/029/2486/0292486.pdf [retrieved on 2015-01-07]**
• **Johna Thomas ET AL: "How Do Different Temperatures Affect the Fermentation Rate of Yeast?", CALIFORNIA STATE SCIENCE FAIR 2003 PROJECT SUMMARY, 31 December 2003 (2003-12-31), XP55402179, Retrieved from the Internet: URL:http://cssf.usc.edu/History/2003/Projects/J1333.pdf [retrieved on 2017-08-29]**

- **H.S. Son ET AL: "A Novel Approach for Estimating Sugar and Alcohol Concentrations in Wines Using Refractometer and Hydrometer", Journal of Food Science, vol. 74, no. 2, 1 March 2009 (2009-03-01), pages C106-C111, XP55257944, US ISSN: 0022-1147, DOI: 10.1111/j.1750-3841.2008.01036.x**

## Beschreibung

Die Erfindung betrifft ein Verfahren und eine Einrichtung zum Steuern einer Gärung. Die Erfindung betrifft weiterhin ein Computerprogramm und ein Computerprogrammprodukt zur Durchführung des Verfahrens.

Bei der alkoholischen Gärung wird Zucker durch Hefe in Alkohol, Kohlendioxid und Gärnebenprodukte, wie Glyzerin, umgewandelt, und zwar unter Wärmeentwicklung. Bei der Herstellung alkoholhaltiger Flüssigkeiten, z.B. alkoholischer Getränke, wie Wein, Bier und Branntwein, ist die Gärung der wichtigste Schritt im Herstellungsprozess. Im Rahmen der Herstellung ist es unumgänglich, diesen Umwandlungsprozess kontinuierlich zu überwachen. Nur auf diese Weise ist es möglich, Störungen im Gärvorgang, wie bspw. einen Gärstopp, frühzeitig zu erkennen und rechtzeitig geeignete Maßnahmen ergreifen zu können. Sicherlich bei mindestens 10 % aller Gärungen ergeben sich Störungen.

Für die Kontrolle der Gärung ist es erforderlich, die Zuckerabnahme - bis zum Restzucker - zu erfassen, möglichst auch die Gärtemperatur - bestenfalls noch die Alkoholzunahme. In einer sogenannten Gärkurve wird über der Zeitachse der Alkoholgehalt und möglichst auch die Gärtemperatur aufgetragen. Anhand des Verlaufs der Gärkurve(n) erkennt der Fachmann, ob der Gärprozess korrekt abläuft.

Die Umwandlung von Zucker in Alkohol ist mit der Gärgleichung von Gay-Lussac beschrieben. Nach dieser werden 100 g Zucker in 47 g Alkohol und 51 g $CO_2$ umgewandelt. Somit kann durch Bestimmen des Zuckergehalts in einem Gärgut auf den Alkoholgehalt rückgeschlossen werden. Dabei ist zu beachten, dass in den verschiedenen Industriebereichen und Weinbauregionen unterschiedliche (Ersatz-)Maßeinheiten für den "Zuckergehalt" verwendet werden, z.B. °Oechsle, °Balling, °Baume, % Brix, °KMW, a.p., °Plato.

Hierbei wird mit dem Begriff Zuckergehalt der gewichtsmäßige Anteil oder die Konzentration in einer Flüssigkeit bezeichnet. Somit wird angestrebt, die genaue Bestimmung eines Gehalts an Zucker in g/l oder in % anzugeben. Analoges gilt für den Alkoholgehalt.

Zur Erstellung von Gärkurven werden unterschiedliche Messgeräte eingesetzt:
Seit einiger Zeit gibt es vollautomatische Messsysteme. Diese messen online indirekt Zucker und Alkohol, z.B. über die Messung des austretenden CO2. Solche Systeme sind sehr teuer. Deshalb werden die Gärkurven meist manuell - wie seither - erstellt, z.B. mittels Aräometers. Weit verbreitet ist der Einsatz von Saccharimetern bei den Brauern und Brennern und sog. Mostwaagen bei Winzern, im deutschsprachigen Raum auch als Oechsle-Waagen bezeichnet. Die verwendeten Aräometer sind Dichtemessgeräte. Mit solchen Aräometern, z.B. mit einer Mostwaage, wird der Zuckergehalt des Gärgutes indirekt bestimmt. Es ist damit keine analytisch genaue Bestimmung möglich, weil in den in Betracht kommenden Flüssigkeiten neben Zucker noch Wasser, Ethanol, Salze, Mineralstoffe und Anderes enthalten ist, und zwar in recht unterschiedlichen und variierenden Konzentrationen. Zwar sind die so gemessenen Werte nicht ganz genau - und werden mit zunehmendem Alkoholgehalt während einer Gärung prinzipiell immer ungenauer. Sie zeigen auf jeden Fall auf, wenn die Gärung Störungen aufweist. Dies ist der Hauptzweck der Erstellung einer Gärkurve. Eine Gärstörung muss baldmöglichst erkannt werden damit rechtzeitig entsprechende Maßnahmen ergriffen werden können. Für eine aräometrische Messung werden 200 - 250 ml Probe benötigt, damit ein entsprechendes Aräometer darin schwimmen kann. Die Flüssigkeit wird in ein Behältnis gefüllt und darin wird das Aräometer eingesenkt. In Abhängigkeit der Dichte sinkt das Aräometer ein. An der Schnittstelle des Aräometerteils, das aus der Flüssigkeit ragt - und in dem eine Skale ist - mit der Flüssigkeitsoberfläche wird der Skalenwert abgelesen. Der Zeitbedarf ist 10 bis 15 Minuten - bei korrekter Durchführung. Dazu müssen mehrere wichtige "Kleinigkeiten" beachtet werden. Am Schluss muss noch der Messwert temperaturkorrigiert werden, weil ein Aräometer auf eine Messtemperatur von 20 °C justiert ist, dies aber selten gegeben ist. Das Messen mit Aräometern ist also umständlich, zeitintensiv und außerdem umso ungenauer je höher der Alkoholgehalt der gärenden Flüssigkeit ist. Die Erfassung des Alkoholgehalts ist mittels Aräometern in gärenden Flüssigkeiten nicht möglich. Deshalb ist in mit Aräometern erstellten Gärkurven keine Alkoholkurve enthalten. Die mittels Aräometers ermittelten Dichtewerten und die evtl. ergänzend gemessenen Temperaturen werden manuell in ein Diagramm auf Papier eingezeichnet. Aufgrund des Zeitaufwandes wird prinzipiell nur ein Messwert pro Tag erstellt.

Hier wird nun eine neue Methode zur manuellen Erstellung von Gärkurven vorgestellt, und zwar mittels nicht stationärer Refraktometer, also sog. Handrefraktometer. Es gibt Hand-Digitalrefraktometer und herkömmliche. Bei Digitalrefraktometern werden 1 - 2 Tropfen Probe auf ein ca. Centgroßes Glasprisma gegeben, der Startknopf gedrückt; 2 - 3 Sekunden später wird der temperaturkorrigierte Messwert digital angezeigt. Bei herkömmlichen Refraktometern werden 3 - 4 Tropfen Probe auf ein Glasprisma gegeben (ca. 2 x Centgroß). Zum gleichmäßigen Verteilen der Probe auf dem Glasprisma wird eine Klappe darauf gelegt. Das Messergebnis wird dann durch ein Okular (ähnliche Konstruktion wie bei einem Fernglas)im Gerät an einer Skale abgelesen; Zeitbedarf ca. 1,5 Minuten. Die Messung mit Refraktometern können also ungleich schneller als Messungen mit Aräometern durchgeführt werden.

Seither gelten Refraktometer als nicht geeignet zur Erstellung von Gärkurven. So wird in dem "Praktikerhandbuch Oenologie" des DLR Rheinpfalz (Neustadt an der Weinstraße), Stand 2008, dargelegt, dass es mit Refraktometern nicht möglich ist, den Restzuckergehalt in gärenden Mosten aus dem abgelesenen Mostgewicht zu ermitteln. Handrefraktometer werden heute vielfach verwendet, um - wie mit Aräometern - indirekt den Zuckergehalt in unvergorenen Flüssigkeiten, wie Traubenmost, zu messen. Man kann Refraktometer mit denselben Skalenmaßen wie Aräometer

EP 2 948 768 B1

bekommen.

In der Refraktometrie wird aus einer Veränderung der Brechzahl auf die Konzentration eines bekannten Probenbestandteils geschlossen. Als Refraktion bzw. Brechung wird jede Änderung der Ausbreitungsrichtung von Wellen beim Übergang aus einem ersten Medium in ein zweites Medium bezeichnet. Die Brechzahl, die auch als Brechungsindex bezeichnet wird, ist eine Materialkonstante für die Ausbreitung des Lichts in einem Medium. Es wird folglich der quantitative Zusammenhang zwischen Brechungsindex und Zucker- bzw. Alkoholgehalt herangezogen.

Die Gärkurvenerstellung mit Refraktometern hat viele Vorteile im Vergleich zur Verwendung von Aräometern. So ist die Zuckerbestimmung viel einfacher als mit einem Aräometer. Ein Refraktometer, das aus stabilem Metall oder Kunststoff besteht, bricht nie entzwei - im Gegensatz zu den aus dünnstem Glas bestehenden Aräometern. Die Messungen können viel schneller und mit ungleich geringeren Probemengen durchgeführt werden als mit Aräometern. Der größte Vorteil des neuen Verfahrens ist, dass es gelungen ist, den Alkoholeinfluss aus den refraktometrischen Messwerten zu eliminieren und den Alkohol selber zu bestimmen, auch wenn mit der Erstellung einer Gärkurve erst begonnen wird nachdem die Gärung bereits eingesetzt hat. Auch mit dieser Methode können keine analytisch genauen Werte der vielen Bestandteile einer gärenden Flüssigkeit bestimmt werden, aber der sehr wesentliche Ethanolgehalt kann ziemlich genau gemessen werden.

Die Druckschrift DE 37 87 515 T2 beschreibt ein Verfahren zur Kontrolle alkoholischer Gärungen, bei dem eine Kontrolle der Gärung durch geeignete Änderungen der Temperatur des Gärungsmediums vorgenommen wird.

Die Druckschrift "A new refractometric methodology used to monitor fermentations", George Cojocaru et al, XP055115712 zeigt ein Verfahren zur Kontrolle alkoholischer Gärungen, bei dem eine Kontrolle der Gärung durch geeignete Änderungen der Temperatur des Gärungsmediums vorgenommen wird.

Die Druckschrift "Muti-purpose optoelectronic instrument for monitoring the alcoholic fermentation of wine", F. Jimenez et a Sensoring Proceedingsl, 2011 IEEE: Limerick, Ireland, XP032093392, beschreibt ein Verfahren zum Überwachen einer alkoholischen Gärung, bei dem eine Dichte eines Gärguts gemessen wird.

Die Druckschrift CN 201 955 301 U zeigt einen Gärtank zur Durchführung einer Gärung.

Die Druckschrift EP 1 559 775 A2 beschreibt ein Verfahren zum kontrollierten Vergären einer Flüssigkeit, bei dem die Temperatur der Flüssigkeit zumindest während eines Teils des Gärprozesses derart geregelt wird, dass ein vorgebbarer zeitlicher Verlauf mindestens einer den Gärzustand charakterisierenden Größe zumindest annähernd eingehalten wird.

Die Druckschrift "The Kinetics of Alcoholic Fermentation of Sugars" von Reginald Haydn Hopkins und Richard Henry Thomas beschreibt das Verhalten zweier Zuckerarten und Temperaturkoeffizienten während einer Gärung.

Die Druckschrift "How do different Temperatures affect the Fermentation Rate of Yeast" von John Thomas und Peter Zellmann beschreibt die Änderung von Gärungsraten in Abhängigkeit von der Temperatur.

Die Druckschrift "A Novel Approach for Estimating Sugar and Alcohol Concentrations in Wines Using Refractometer and Hydrometer" von H.S. Son, Y.S. Hong, W.M. Park, M.A. Yu und C.H. Lee stellt eine Formel zur Berechnung von Restzucker und Alkohol vor.

Vor diesem Hintergrund werden ein Verfahren mit den Merkmalen des Anspruchs 1, eine Einrichtung gemäß Anspruch 11 und ein Computerprogramm mit den Merkmalen des Anspruchs 12 sowie ein Computerprogrammprodukt nach Anspruch 13 vorgestellt.

Es wird ein Verfahren zum Steuern einer Gärung eines Gärgutes beschrieben, bei dem ein Gärverlauf überwacht und eine Temperatur und die Zuckerabbaurate des Gärgutes ermittelt wird, wobei durch Beeinflussung der Temperatur des Gärgutes der Gärverlauf gesteuert und/oder geregelt wird. Es wird somit eine Regelung der Gärung bzw. eine Regelung des Gärverlaufs vorgenommen. Dabei wird durch Beeinflussen, d. h. durch Erhöhen oder Absenken, der Temperatur des Gärguts der Gärverlauf beeinflusst. Wird erkannt, dass der Gärverlauf nicht so wie gewünscht verläuft, kann so schnell durch Beeinflussen der Temperatur reagiert werden. Eine Erhöhung oder Absenkung der Temperatur wirkt sich nämlich auf den Gärverlauf aus. Zum Ermitteln des Gärverlaufs wird typischerweise in regelmäßigen Abständen der Zucker- und gegebenenfalls auch der Alkoholgehalt des Gärgutes bestimmt.

Die Temperatur des Gärguts kann mit einem Thermometer gemessen werden, das über eine Funkeinrichtung verfügt, so dass die Temperatur zu einer Basisstation übermittelt werden kann, an der unter Berücksichtigung des Gärverlaufs die Beeinflussung der Temperatur zur Regelung des Gärverlaufs bewirkt wird. Die Informationen zum Gärverlauf kann an die Basisstation ebenfalls per Funkübertragung erfolgen. Es kann auch vorgesehen sein, dass die Informationen zum Gärverlauf, also Zucker- bzw. Alkoholgehalt, an das Thermometer übergeben wird, das dann diese Informationen zusammen mit der Temperatur an die Basisstation übergibt.

Bei dieser Basisstation kann dann eine Einrichtung zum Überwachen einer Gärung eines Gärgutes, insbesondere zur Durchführung eines vorstehend beschriebenen Verfahrens, vorgesehen sein, mit einer Regeleinrichtung, die dazu eingerichtet ist, einen Gärverlauf des Gärgutes durch Beeinflussung der Temperatur des Gärgutes zu regeln.

Es wird ein weiteres Verfahren zum Überwachen einer Gärung eines Gärgutes beschrieben, bei dem in zeitlichen Abständen mittels eines insbesondere portablen Refraktometers ein Brechungsindex des Gärgutes gemessen wird und auf Grundlage dieser Messung ein aktueller Zuckergehalt des Gärgutes bestimmt wird, wobei unter Berücksichtigung eines Anfangswerts für den Zuckergehalt aus dem aktuellen Zuckergehalt ein aktueller Alkoholgehalt bestimmt wird und auf diese Weise eine Gärkurve erstellt wird. Dieses Verfahren, das insbesondere zur Bestimmung des Zucker- bzw. Alkoholgehalts des Gärgutes dient, kann gesondert oder in Kombination mit dem zuvor beschriebenen Verfahren nach Anspruch 1 und/oder 2 zur Anwendung kommen. Das Verfahren zum Bestimmen des Alkoholgehalts kann somit auch unabhängig von der Regelung des Gärverlaufs erfolgen.

Der aktuelle Zuckergehalt kann mit folgender Formel berechnet werden:

$$ABK = AB * K0\_Fak + SB * (1 - K0\_Fak),$$

wobei SB der Anfangs-Zuckerwert in Brix bei Most in unvergorenem Zustand, AB der aktuelle Zuckerwert in Brix, ABK der korrigierte aktuelle Zuckerwert in Brix und K0_Fak ein Umrechnungsfaktor ist. Anstelle der Brix-Werte mönnen auch nD-Werte (nD: Brechungsindex) verwendet werden.

Der Umrechnungsfaktor kann als nicht konstant angenommen werden, wobei dieser mit fortlaufender Gärung zunehmen kann. Dies bedeutet, dass zu Beginn der Gärung der Umrechnungsfaktor geringer als zum Ende der Gärung ist. Als Wert für den Umrechnungsfaktor hat sich etwa 1,30 als geeignet herausgestellt.

In Ausgestaltung werden die refraktometrischen Messwerte, z.B. manuell per Eintippen in eine Tastatur, an eine Basisstation übermittelt. Diese Basisstation stellt die empfangenen Ergebnisse dar und interpretiert sie. Die Darstellung der Ergebnisse kann alternativ oder ergänzend von dem Refraktometer vorgenommen werden.

Das vorgestellte Verfahren ermöglicht ein teilautomatisiertes, effizientes Überwachen des Gärvorgangs eines Gärgutes, bspw. eines Mostes bei der Weinherstellung, mittels Gärkurven, die ohne großen zeitlichen Aufwand für den Nutzer erstellt werden können. Durch die auf diese Weise erreichte hohe Sicherheit bei der Gärung können Produkte hoher Qualität hergestellt werden.

Es ist zu beachten, dass Gärkurven von jeder Gärung erstellt werden sollten, da auf diese Weise Gärstörungen sehr schnell erkannt werden können. Auf diese kann dann auch sehr schnell reagiert werden.

Durch die Verwendung des Refraktometers reduziert sich der Zeitaufwand für die Messung erheblich, auf etwa 30 Sekunden wobei nur 1 bis 2 Tropfen Probe für jede Messung benötigt werden.

Aus den Messwerten berechnet die verwendete Software z.B. die annähernd richtigen Oechslewerte und zwar, alkohol- und temperaturkorrigiert, die richtigen Gärkurven in bspw. °KMW oder °Oechsle sowie korrekte Alkohol- und Temperaturkurven auch unter Berücksichtigung einer eventuellen Anreicherung und den ungefähren Restzucker bei der Endvergärung.

Die Durchführung der Messung nach dem vorgestellten Verfahren stellt sich als sehr einfach und bequem dar. Deshalb kann auch unter Zeitdruck eine Gärkurve bzw. ein Gärdiagramm mit minimalem Aufwand erstellt werden. Die Kurven können laufend und automatisch erstellt und ausgedruckt werden. Alles ist in der Entwicklung an einem Bildschirm sichtbar zu machen. Zur besseren Information können auch Übersichten mit den wichtigsten Daten von laufenden Gärungen erstellt werden.

Die vorgestellte Messeinrichtung umfasst typischerweise ein Refraktometer, üblicherweise ein Digital-Refraktometer, und ggf. eine Basisstation. Diese können jeweils über eine Schnittstelle für eine drahtlose Kommunikation, üblicherweise eine drahtlose Funkverbindung, verfügen. Zur Überwindung von Übertragungsschwierigkeiten kann ein ebenfalls mit Schnittstellen versehenes Smartphone zur Zwischenspeicherung der Messwerte eingesetzt werden.

Das vorgestellte Computerprogramm umfasst Programmcodemittel zur Durchführung eines Verfahrens der vorstehend beschriebenen Art. Hierzu wird das Computerprogramm auf einer Recheneinheit zur Ausführung gebracht. Diese Recheneinheit kann in einem im Rahmen des Verfahrens verwendeten Messeinrichtung vorgesehen sein. Dabei kann eine Recheneinrichtung in dem Refraktometer und/oder eine Recheneinrichtung in der Basisstation zur Anwendung kommen.

Von Bedeutung ist, dass mit dem Refraktometer der Zucker- und damit der Alkoholgehalt eines Gärgutes schnell bestimmt werden kann. Über eine Kennung, bspw. einen Code, oder Einsatz eines Transponders kann das Ergebnis der Messung mit vormaligen und zukünftigen Messungen desselben Gärgutes in Verbindung gebracht werden. Diese Zuordnung kann auch durch Eingabe eines das Gärgut kennzeichnenden Angabe durch den Nutzer erfolgen.

In einer Ausführung wird mit dem Refraktometer zunächst ein Code am Tank des Gärgutes eingelesen und anschließend die eigentliche Messung durchgeführt. Auf diese Weise kann die Zuordnung von Messwert zu Gär- bzw. Messgut erfolgen. Das Ergebnis der Messung kann in dem Refraktometer selbst ausgewertet werden oder zu einer Basisstation übertragen werden. Eine sich ergebende Gärkurve kann ebenfalls direkt am Refraktometer und/oder an der Basisstation angezeigt werden. Wird ein problematischer Messwert erkannt, so kann dies am Refraktometer und/oder an der Baisstation angezeigt werden. Geeignete Maßnahmen zur Behebung der Störung können dann ausgelöst durch ein Signal des Refraktometers und/oder der Basisstation an eine zentrale Steuerung ausgesendet werden. Die Basisstation kann Komponente einer übergeordneten Steuerungs- und Regeleinrichtung sein.

Das mobile Refraktometer und die Basisstation, die auch als Empfangsstation bezeichnet werden kann, können über eine drahtlose Funkverbindung zur Kommunikation bzw. zum Datenaustausch miteinander verbunden sein. Diese stellt eine bidirektionale Verbindung dar.

Alternativ kann zur automatischen Datenübermittlung ein Smartphone eingesetzt werden. Smartphones werden in großer Anzahl produziert und können deshalb relativ preiswert bezogen werden. Diese verfügen in den meisten Fällen über eine Bluetooth- oder WLAN-Schnittstelle oder über eine anderen entsprechende Schnittstelle zum Empfang und zur Übertragung drahtloser Nachrichten sowie einen Datenspeicher. Zudem sind sie beschriftbar. Aus diesen Gründen sind sie für eine automatische Datenübertragung gut geeignet.

Ein Beispiel für eine mögliche Anwendung wird nachfolgend gegeben. Während der Weinlese wird der Most bzw. die Maische aus den geernteten Trauben zu Wein vergoren. Zur Beobachtung der Gärung werden Gärkurven erstellt. Meist werden dazu Dichtemessgeräte verwendet, bspw. Aräometer, seltener Biegeschwinger. Die gemessenen Werte repräsentieren den vorhandenen vergärbaren Zucker. Die gemessenen Werte werden in ein Achsenkreuz eingezeichnet. Dabei ist auf der Y-Achse die Skala für den Zucker, auf der X-Achse wird das Datum der Messung eingetragen. Vor Gärbeginn ist der Zuckergehalt am höchsten. Dieser nimmt im Verlauf der Zeit ab. Deshalb verlaufen die Gärkurven von oben schräg nach unten.

Für eine Messung mit einem Aräometer werden ca. zehn Minuten benötigt und 200 bis 250 ml Flüssigkeit. Zur automatischen Übertragung der Messwerte an eine unter Umständen weit entfernte Datenverarbeitungsanlage als Basisstation, bspw. ein PC oder ein Laptop, eventuell in einem anderen Gebäude, benötigt man ein Smartphone, das vorteilhafterweise dieselbe Schnittstelle für drahtlose Übertragung von Daten besitzt wie die Basisstation. Außerdem benötigt man ein Refraktometer anstelle der Dichtemessgeräte, möglichst mit einer Bluetooth- bzw. WLAN-Schnittstelle oder einer vergleichbaren Schnittstelle. Am besten eignet sich ein kleines Mini-Digitalrefraktometer. Dieses Gerät ist handlich und misst zuverlässig, ist batteriebetrieben und hat eine beleuchtete Digitalanzeige, so dass auch bei schlechten Lichtverhältnissen die Messergebnisse gut ablesbar sind.

Die refraktometrischen Messergebnisse repräsentieren die Zuckerkonzentration. Zweckmäßigerweise wird direkt am Gärtank gemessen, bspw. in °Oechsle, %Brix usw. Eine Messung dauert ca. 20 Sekunden. Man benötigt etwa zwei Tropfen Most oder eine entsprechende Menge Maischeflüssigkeit bzw. -brei. Der Messwert kann in das Zahlenfeld des Smartphones eingetippt werden, ebenso die Tankbezeichnung zur Zuordnung des Messwerts zum zugehörigen Tank. Falls erwünscht, kann auch die Gärtemperatur, die an einem Thermometer am Gärtank angezeigt wird, eingetragen werden. Befindet sich am Gärtank oder in der Nähe ein Barcode, kann dieser zur Identifizierung der Messwerte mit dem Smartphone abgelesen werden. Ist ein Transponder am Tank befestigt, kann dies alternativ verwendet werden, da der Transponder zur Identifizierung der Messwerte die Tankbezeichnung drahtlos an das Smartphone überträgt.

Sofern das Refraktometer eine entsprechende Funkschnittstelle hat, werden die Messwerte automatisch zum Smartphone übertragen. Das Smartphone kann dabei in der Tasche bleiben. Sofern das Refraktometer keine Schnittstelle besitzt, kann das Messergebnis mittels im Smartphone integrierter Kamera fotografiert und gespeichert werden. Analog gilt dies auch für die Thermometeranzeige der Gärtemperatur. Da im Smartphone eine Uhr und ein Kalender integriert sind, können die Messwerte zeitlich zugeordnet werden.

Nach der schnellen Messung und Übertragung der Daten von einem Gärtank oder von mehreren an ein Smartphone können die Daten im Smartphone zur datenverarbeitenden Anlage, der Basisstation, per Schnittstelle RS 232 und Kabel oder drahtlos über Funk übertragen werden. Dazu muss der Smartphoneträger in der Nähe der Basisstation sein. Dadurch werden Übertragungsfehler vermieden, die bei direkter Funkübertragung vom Refraktometer am Tank zur Basisstation bei Gärkellern meist auftreten, da viele Einrichtungen aus reflektierendem Edelstahl bestehen und ggf. dicke, für Funk-übertragungen undurchdringliche Eisenbetonwände und -decken vorhanden sind. Aus den Daten kann die Basisstation automatisch eine Gärkurve erstellen und weitere Auswertungen vornehmen. Das Smartphone kann über eine APP für die vorzunehmenden Funktionen programmiert werden. Dies gilt auch für Gärungen in Brauereien sowie Brennereien. Es kann alternativ eine Übertragung per Funk oder per Hand an ein Thermometer erfolgen, dass die Daten zusammen mit Temperaturwerten an die Basisstation überträgt, um eine Regelung des Gärverlaufs durch Beeinflussen der Temperatur des Gärgutes vorzunehmen.

Das vorgestellte Verfahren zur Übertragung von Ergebnissen bzw. Messwerten bei einer Gärüberwachung kann grundsätzlich zum Übertragen von Messwerten eingesetzt werden, bei dem ein Smartphone eingesetzt wird. Daher wird hier ein Verfahren zum Übertragen von Messwerten vorgestellt, bei dem die Messwerte an der Messstelle an das Smartphone übertragen werden, von diesem gespeichert werden und anschließend von dem Smartphone an eine Basisstation, die entfernt von der Messstelle sich befinden kann, übertragen werden. Auf diese Weise ist eine sichere Übertragung der Daten unabhängig von den Umgebungsbedingungen möglich. So ist es möglich, dass der Nutzer zunächst an der Messstelle die Messwerte mit dem Smartphone aufnimmt und anschließend sich zu einer Basisstation bewegt, bei der die Messwerte von dem Smartphone auf die Basisstation übertragen werden.

Weiterhin ist der Einsatz einer sogenannten Dropbox möglich. Hierbei werden die erfassten Daten der Basisstation entweder per E-Mail als Dateianhang zur Verfügung gestellt oder über einen Dropboxdienst in einen vorgegebenen Ordner des PCs geschrieben. Die komplette Weiterverarbeitung erfolgt dann am PC bzw. an der Basisstation.

Auf diese Weise sind die Möglichkeiten gegeben, ohne schwer bedienbare Kabel oder zusätzliche Hardware mit einer breiten Basis von PCs zu synchronisieren. In beiden Fällen ist die Mindestanforderung ein PC und ein Internetanschluss.

Auch in diesem Fall kann ein Smartphone eingesetzt werden. Es erfolgt dann zunächst die Messwerterfassung, wobei eine Behälternummer mittels Einlesens eines Codes oder einer optionalen manuellen Eingabe eingegeben werden. Bei wiederholter Eingabe innerhalb eines bestimmten Zeitraums kann der zuvor erfasst Messwert überschrieben werden. Auf dem Smartphone kann die Behälternummer sowie der Messwert angezeigt werden. Es kann auch eine Fehlermeldung erfolgen, wenn der bzw. die Messwerte zu stark abweichen. Optional kann die Behältertemperatur bzw. die Temperatur des Gärgutes eingegeben bzw. eingelesen werden. Außerdem können Datum und Uhrzeit erfasst oder eingegeben werden.

Bei Gärprozessen in der Weinherstellung ist es elementar wichtig, einen gleichmäßigen, möglichst schonenden Gärverlauf zu erzielen. Es ist also erwünscht, einen möglichst gleichmäßigen Zuckerabbau des Mostes über die Gärzeit einzuhalten. Diese Zuckerabbaurate (Gärrate), zum Beispiel in Brix pro Zeiteinheit, wird vom Kellermeister oder Winzer vorgegeben und soll durch geeignete immer wiederkehrende Messvorgänge und entsprechende Stelleingriffe in den Gärprozess erzielt werden.

Als geeignete Messgröße wird sinnvollerweise eine Refraktometermessung verwendet, wobei jede, den aktuellen Zuckergehalt des Weinmostes bestimmende Messgröße, alternativ verwendet werden kann.

Aus den regelmäßigen bzw. zyklischen Messungen des aktuellen Zuckergehalts einer laufenden Gärung lassen sich durch Differenzbildung und Normierung über der Zeit eine geeignete Zuckerabbaurate $R_Z$ bestimmen. Diese momentante Abbaurate $R_{Z\text{-}Ist}$ ist auf eine vom Winzer bzw. Kellermeister gewünschte Soll-Abbaurate $R_{Z\text{-}Soll}$ einzustellen, in dem man die Gärtemperatur im Gärbehälter durch geeignete regelungstechnische Maßnahmen auf einen gewünschten vorgegebenen Verlauf einstellt.

Der Temperatur-Sollwert der Gärtemperaturregelung im Gärbehälter wird durch einen geeigneten Algorithmus ermittelt.

Die Idee der Berechnung einer geeigneten Solltemperatur basiert aus der bekannten Arrhenius-Gleichung:

$$k = A \cdot e - \frac{E_A}{R \cdot T}$$

A    präexponentieller Faktor oder Frequenzfaktor, entspricht nach der Stoßtheorie dem Produkt aus der Stoßzahl Z und dem Orientierungsfaktor P

$E_A$    Aktivierungsenergie (Einheit: J·K-1)

R    universelle Gaskonstante (8,314 J·K-1·mol-1)

T    absolute (thermodynamische) Temperatur (Einheit: K)

k    Reaktionsgeschwindigkeitskonstante

Die vereinfachte Form dieser Arrhenius-Gleichung bildet die van't Hoff'sche Regel.

Die van't Hoff'sche Regel, auch RGT-Regel (Reaktionsgeschwindigkeit-Temperatur-Regel) ist eine Faustregel der chemischen Kinetik und erlaubt die Abschätzung vieler Phänomene der Chemie, Biochemie und Ökologie. Sie besagt, dass chemische Reaktionen bei einer um 10 K erhöhten Temperatur doppelt bis viermal so schnell ablaufen.

Der Faktor, um den die Reaktionsgeschwindigkeit konkret steigt, wenn die Temperatur um 10 K erhöht wird, heißt Q10-Wert.

$$Q_{10} = \left(\frac{R_2}{R_1}\right)^{\frac{10K}{T_2-T_1}}$$

wobei R1 und R2 die jeweilige Reaktionsgeschwindigkeit bei Temperatur T1 bzw. T2 bezeichnet.

Setzt man $R_1 = R_{Z\text{-}Ist}$ bei Temperatur T1 und $R_2 = R_{Z\text{-}Soll}$ bei einer zukünftigen Temperatur T2, so lässt sich die notwendige Temperaturkorrektur $T_K = T_2\text{-}T_1$ aus der obigen Gleichung leicht errechnen und somit eine neue Solltemperatur einstellen.

In der Praxis ist $Q_{10}$ = 2-4 erwartbar. Setzt man nun für $Q_{10}$ = 3, einen mittleren Wert an, so kann man einen realistischen Temperaturkorrekturwert berechnen, der dem exakten Wert nahekommt.

Ein realistischer Schätzwert für $Q_{10}$ lässt sich auch auf geeignete Weise aus den unterschiedlichen Zucker-Abbauraten bei zwei möglichst unterschiedlichen Temperatur-Arbeitspunkten aus der vorstehend genannten Gleichung berechnen.

Es wird somit ein Verfahren gemäß Anspruch 1 erläutert, das zum Steuern einer Gärung geeignet ist.

Eine Einrichtung zur Durchführung des Verfahrens wird ebenfalls vorgestellt.

Dieses Verfahren ermöglicht einen höheren Automatisierungsgrad, wobei ein Thermometer, insbesondere ein Thermometer mit einer Digitalanzeige oder ein Digital-Thermometer, verwendet wird, das über eine Funkeinrichtung verfügt. Der Messwert des Refraktometers wird an das Thermometer gegeben, das diesen typischerweise mit dem gemessenen Temperaturwert an eine Basisstation gibt. Alternativ können sowohl das Refraktometer und das Thermometer die gemessenen Werte an die Basisstation übermitteln.

Der Zusammenhang zwischen der Änderung der Temperatur und der Änderung der Gärkurve kann empirisch ermittelt werden. Eine lineare Gärkurve ist ideal, da bei dieser der Alkoholverlust durch ausströmendes $CO_2$ minimiert ist.

Somit kann über eine Steuer- und/oder Regeleinrichtung, die der Basisstation zugeordnet ist, die Temperatur im Tank gesteuert bzw. geregelt werden, um so Einfluss auf den Gärverlauf zu nehmen.

Bei dem Verfahren erfolgt bspw. eine Messung des Extrakts bzw. Mostgewichts des Mostes bzw. der Maische am Gärbehälter in unvergorenem Zustand, d. h. ohne Alkohol, mittels eines Handfraktometers, insbesondere eines digitalen Handfraktometers, und eine Übermittlung der Messergebnisse mittels eine Funkeinrichtung, bspw. eines WLAN, im Refraktometer an ein am Tank befestigtes Thermometer, insbesondere eines digitalen Thermometers, mit Funkeinrichtung oder direkt, bspw. mittels eines Repeaters, an eine Recheneinheit, bspw. einen PC oder Laptop, typischerweise bei der Basisstation.

Da im Gärgut neben Wasser und vergärbarem Zucker im Durchschnitt ca. 2 % nicht gärfähiges Material vorhanden ist und dieses Material das refraktometrische Messergebnis beeinflusst, ist mit einem durchschnittlichen Zuckermessfehler von ebenfalls etwa 2 % zu rechnen.

Zu der Recheneinheit werden von dem Thermometer die zum gesendeten Refraktometer-Messwert zugehörige Tankbezeichnung, die Ist-Temperatur und die eingestellten Regeltemperaturwerte gesendet. Anschließend erfolgen, bspw. täglich, weitere Refraktometer-Messungen. Diese im weiteren Gärverlauf zunehmend alkoholverzerrten Messdaten samt den Daten des Thermometers, nämlich Ist-Temperatur, Tankbezeichnung, eingestellte Regeltemperaturwerte, werden von der Recheneinheit angefordert bzw. dorthin gesendet. Ein Computerprogramm bzw. eine Software der Recheneinheit berechnet in Ausgestaltung laufend, ob bzw. wie stark die eingestellten Temperaturregelwerte geändert werden müssen, damit der gewünschte möglichst geradlinige Gärkurvenverlauf erreicht wird und verändert, falls dies erforderlich ist, automatisch per Funk die eingestellten Regeltemperaturwerte.

Durch einen geradlinigen Gärkurvenverlauf wird der Aromaverlust durch den $CO_2$-Austritt minimiert. Von der Software werden aus dem alkoholverzerrten Refraktometer-Messwert der alkoholkorrigierte Extrakt und der erreichte Alkoholgehalt berechnet. Die richtigen Messwerte werden zum Thermometer gesendet und an einer Anzeige bzw. einem Display angezeigt, ebenso wie die neuen Regeltemperaturwerte und Alarmnachrichten, wie z. B. ein Gärstopp oder ein außergewöhnliches Gärtemperaturverhalten.

Außerdem wird dort die von der Software berechneten aktuellen und vorherigen Messwerte und die daraus erstellten Gärkurven angezeigt. Die Nutzer, als bspw. die Winzer, Brenner, Brauer usw., erhalten somit am jeweiligen Gärbehältnis bzw. Tank höchst aktuell die Mitteilung, wie es um die Gärung steht.

Dies bedeutet, dass keine Gärkurve mehr per Hand erstellt werden muss. Der Zeitaufwand pro Messung am Gärbehälter beträgt ca. 20 Sekunden, bei 100 Behältern sind dies 2000 Sekunden und somit etwa 33 Minuten insgesamt. Der Zeitaufwand durch Verwendung einer derzeit üblichen Mostwaage zur Gärkontrolle beträgt ca. 8 Minuten. Bei 100 Behältern sind dies 800 Minuten, das entspricht etwa 13 Stunden.

Gärkurven und angefallene detaillierte Gärdaten werden von der Software gespeichert bzw. erstellt und können z. B. an Druckern, die an der Recheneinheit angeschlossen sind, ausgedruckt werden. Die Daten können auch an weitere angeschlossene Geräte übertragen werden.

Das für das Verfahren erforderliche Thermometer, insbesondere Digital-Thermometer, kann in Ausführungsformen einige oder alle der folgenden Eigenschaften haben:

- elektrisch bspw. mit Batterie betrieben,

- spritzwassergeschützt, da es in Gärkellern "nass" ist,

- mit einem aus dem Gehäuse ragenden Fühler versehen,

- mit einem in einem Rohr befindlichen Fühler,

- mit einer Befestigungsmöglichkeit des Fühlers bzw. Thermometers am Tank, z. B. an einer in einen gas- oder flüssigkeitsbefüllten Behälter ragenden und dort befestigten Hülse, bspw. mittels einer Überwurfmutter an einem Gewinde des Hülsenanfangs am Behälter,

- mit einer verstellbaren Regeleinrichtung zur Temperierung des Tankinhalts, bspw. mittels flüssigkeitsdurchflossener Tauchplatten im Behälter oder sogenannte pillow plates, durch Berieselung des Behälters, durch elektrisch betriebene Teile bzw. Geräte, usw. Die Regeleinrichtung ist zur Beeinflussung der Temperatur des Gärgutes mit Kühl- und Wärmegeräten verbunden, verändert die Wirkung der angeschlossenen Geräte, z. B. werden Magnetventile betätigt, d. h. geöffnet oder geschlossen, und so der Durchfluss von Kühlwasser bzw. Warmwasser in Tauchplatten verändert,

- mit Digitalanzeige der Ist-Temperatur, der Regel-Temperatur(en) und weitere Daten,

- mit Funkeinrichtung, bspw. mit der weltweit zugelassenen 2,4 GHz-Frequenz, wie bspw. WLAN oder dergleichen, zur externen Abfrage der Behälter-Bezeichnung, der Ist-Temperaturen, der alkoholverzerrten Messwerte und zur Veränderung der Regeltemperaturen per Funk sowie zur Anzeige der von der Software ermittelten wichtigen Daten.

Das beschriebene Verfahren hat, zumindest in einigen der Ausführungen, erhebliche Vorteile. Die für jede Gärung, sofern gute Qualität des zu produzierenden alkoholischen Getränks erzielt werden soll, unverzichtbare Gärkontrolle und Gärsteuerung können mittels eines solchen Thermometers und einer Organisation, wie dies beschrieben ist, zumindest nahezu vollständig automatisiert werden. Dies kann sehr preiswert in Vergleich zu anderen Gärkontroll- und Gärsteuerungsvorrichtungen erreicht werden.

Weitere Vorteile und Ausgestaltungen der Erfindung ergeben sich aus der Beschreibung und der beiliegenden Zeichnung. Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand von Ausführungsformen in der Zeichnung schematisch dargestellt und wird nachfolgend unter Bezugnahme auf die Zeichnung ausführlich beschrieben.

Figur 1    zeigt in schematischer Darstellung eine Ausführungsform der beschriebenen Messeinrichtung.

Figur 2    zeigt in einem Flussdiagramm einen möglichen Ablauf des vorgestellten Verfahrens.

Figur 3    zeigt Kurven.

Figur 4    zeigt eine Übersicht aller laufenden Gärungen.

Figur 5    zeigt eine Ausführung der Einrichtung zum Überwachen der Gärung.

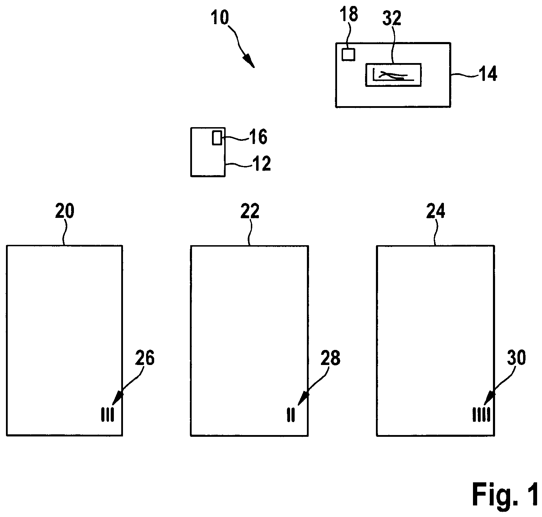

Figur 1 zeigt stark vereinfacht eine Ausführung der vorgestellten Messeinrichtung, die insgesamt mit der Bezugsziffer 10 bezeichnet ist. Diese Messeinrichtung 10 umfasst ein Refraktometer 12 und eine Basisstation 14, die miteinander kommunizieren. Hierzu verfügt das Refraktometer 12 über eine erste Schnittstelle 16 und die Basisstation 14 über eine zweite Schnittstelle 18. Mittels dieser Schnittstellen 16 und 18 erfolgt eine drahtlose Kommunikation.

Weiterhin zeigt die Darstellung einen ersten Gärtank 20, einen zweiten Gärtank 22 und einen dritten Gärtank 24. In diesen ist jeweils ein Gärgut enthalten. Mit dem Refraktometer 12 kann nunmehr das Gärgut jedes Gärtanks 20, 22 bzw. 24 untersucht werden. Um eine Zuordnung zu Messergebnissen früherer und zukünftiger Messungen zu vereinfachen, können die Gärtanks 20, 22 und 24 jeweils mit einem Code 26, 28 bzw. 30 gekennzeichnet sein.

Vor, während oder nach der Messung des Gärgutes eines Gärtanks 20, 22 bzw. 24 wird dann der entsprechende Code 26, 28 bzw. 30, zweckmäßigerweise mit dem Refraktometer 12, eingelesen. Alternativ kann ein Smartphone verwendet werden.

Die ermittelten Messergebnisse werden über die drahtlose Funkverbindung zu der Basisstation 14 übertragen. Bei dieser kann eine Anzeige 32 zur Darstellung der Ergebnisse, typischerweise der Gärkurven, vorgesehen sein.

Der Nutzer kann die Gärtanks 20, 22 und 24 grundsätzlich in beliebiger Reihenfolge untersuchen. Diese kann ihm aber auch vorgegeben werden, bspw. in Abhängigkeit ermittelter Ergebnisse oder auch abgelaufener Zeiträume seit der letzten Messung. Bei außergewöhnlichen Ergebnissen kann der Nutzer auch aufgefordert werden, bestimmte Messungen zu wiederholen, um mögliche Messfehler zu korrigieren.

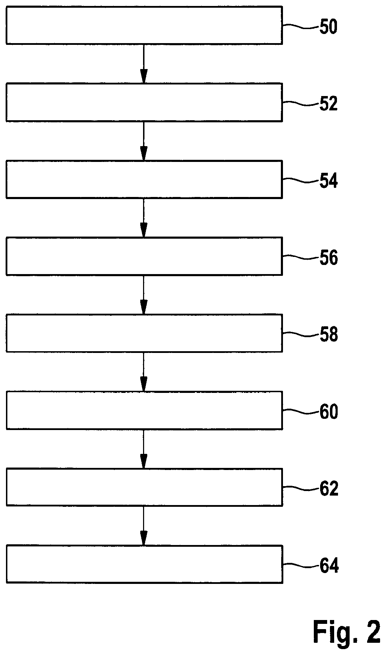

Figur 2 zeigt in einem Flussdiagramm eine mögliche Ausführung des Verfahrens zum Bestimmen des Alkoholgehalts, das automatisiert, typischerweise softwaregesteuert, durchgeführt wird.

In einem ersten Schritt 50 wird das Verfahren gestartet. Es erfolgt in einem nächsten Schritt 52 die Auswahl eines Gärtanks mit enthaltenem Gärgut. Der Nutzer liest in einem darauffolgenden Schritt 54 mit einer Leseeinheit, die dem Refraktometer und/oder der Basisstation zugeordnet ist, einen den betreffenden Gärtank kennzeichnenden Code ein.

Anschließend erfolgt (Schritt 56) die Entnahme einer erforderlichen Menge des Gärgutes und die Messung des Brechungsindexes mit dem Refraktometer. Auf Grundlage des gemessenen Brechungsindexes wird der Zuckergehalt in einem nächsten Schritt 58 berechnet. Dies führt unter Berücksichtigung eines Anfangswerts des Zuckergehalts zur Berechnung des aktuellen Alkoholgehalts des Gärgutes in einem nächsten Schritt 60.

Dieser ermittelte Alkoholgehalt wird mittels drahtloser Funkverbindung zu der Basisstation übermittelt (Schritt 62). Diese führt anhand älterer Messergebnisse eine Plausibilisierung des Messwerts durch und stellt diesen, ggf. nach erfolgreicher Plausibilisierung, in einer Gärkurve dar (Schritt 64).

Für die Berechnung des Zuckergehalts und des Alkoholgehalts können die nachstehend aufgeführten Formeln verwendet werden.

Für die Zuckerkorrektur wurde folgender Zusammenhang ermittelt:

$$ABK = SB - (SB - AB) * K0\_Fak$$
$$= AB * K0\_Fak + SB * (1 - K0\_Fak)$$

wobei:

SB: Start-Brixwert
AB: aktueller Brixwert
ABK: korrigierter aktueller Brixwert
K0_Fak: Umrechnungsfaktor, dieser beträgt in etwa 1,30, ist jedoch nicht konstant

Funktionaler Zusammenhang zwischen Brix und Zuckergehalt: Brix -> Zuckergehalt [g/l]: Z = f(B)

Die Umkehrfunktion:
Zuckergehalt -> Brix : B = f$^1$(Z)

Wenn beim Start des Verfahrens sich bereits Alkohol in der Maische gebildet hat, muss dann entweder der genaue Zuckergehalt oder der genaue Alkoholgehalt durch andere Laborverfahren bestimmt werden.

Ist der Zuckerwert bekannt (in diesem Fall ABK) so folgt:

$$SB = (ABK - AB * K0\_Fak) / (1 - K0\_Fak)$$

Man kann dann die Ausgangformel verwenden.

Ist dagegen nur die Alkoholkonzentration (SA[Vol%]) bekannt, kann ebenfalls auf den Startzucker zurückgerechnet werden:

$$ZZ[g/l] = SA[Vol\%] * 16,4391$$

ZZ: Zuckervergärung zum Bilden des Alkohols
SA: Startalkohol in [Vol%]

$$f(SB) - f(AB*K0\_Fak+SB*(1-K0\_Fak)) = SA * 16,4391$$

Dies lässt sich beispielsweise iterativ nach SB auflösen. Auch dann kann wieder die Ausgangsformel verwendet werden. Ist der aktuelle korrigierte Zuckergehalt aus der Ausgangsgleichung bekannt, kann der aktuelle Alkoholgehalt folgendermaßen berechnet werden (Gärgleichung von Gay-Lussac):

$$Alkohol [Vol\%] = K1\_Fak + (SB - ABK)$$

wobei K1_Fak: Umrechnungsfaktor = 0,61

In einer weiteren Ausführung lässt sich dies genauer berechnen:

```
Alkohol [Vol%]=(K1_Fak-SB/Max_Brix*K2_Fak)*(SB-ABK)
```

wobei K1_Fak: Umrechnungsfaktor = 0,61+K2_Fak/2 = 0,66
K2_Fak: Umrechnungsfaktor = 0,1
Max_Brix:35

Zu beachten ist:
Wenn Alkohol > MAX_ALKOHOL, dann Alkohol = MAX_ALKOHOL

Auf diese Weise wird berücksichtigt, dass die natürliche Gärung bei MAXALKOHOL stoppt. Außerdem wird berücksichtigt, dass bei hohen Start-Brixwerten die Umwandlung in Alkohol nicht so effizient ist wie bei niedrigen Start-Brixwerten.

Der Restzucker (ABK <= 7) berechnet sich zu:

```
Restzucker [g/l] = (ABK - 2) * 10
```

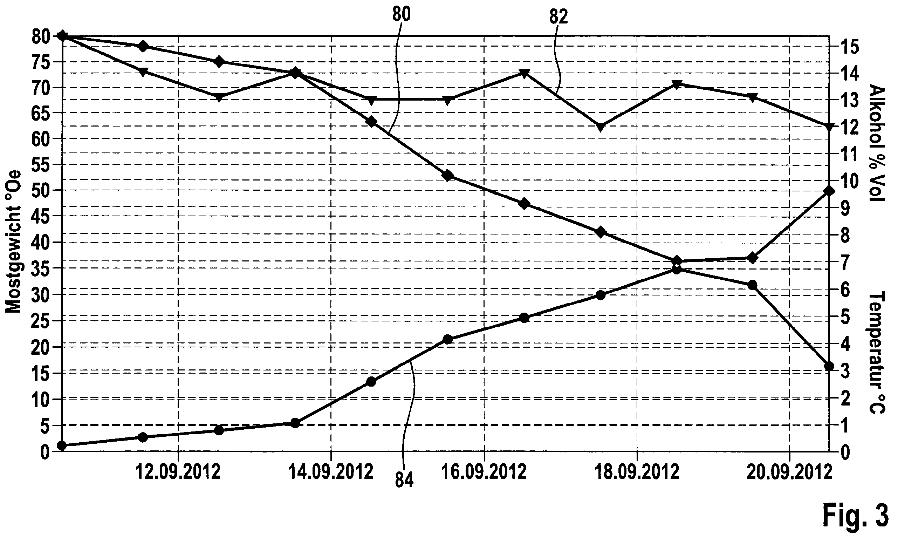

In Figur 3 sind in einem Graphen Gärkurven dargestellt, nämlich der Verlauf des Mostgewichts 80, der Verlauf der Tanktemperatur 82 und der Verlauf des Alkoholgehalts 84.

Mit dem vorgestellten Verfahren können computerunterstützt und für den Anwender erheblich vereinfacht solche Gärkurven erstellt werden. Mit diesen kann dann der Ablauf des Gärprozesses überwacht werden.

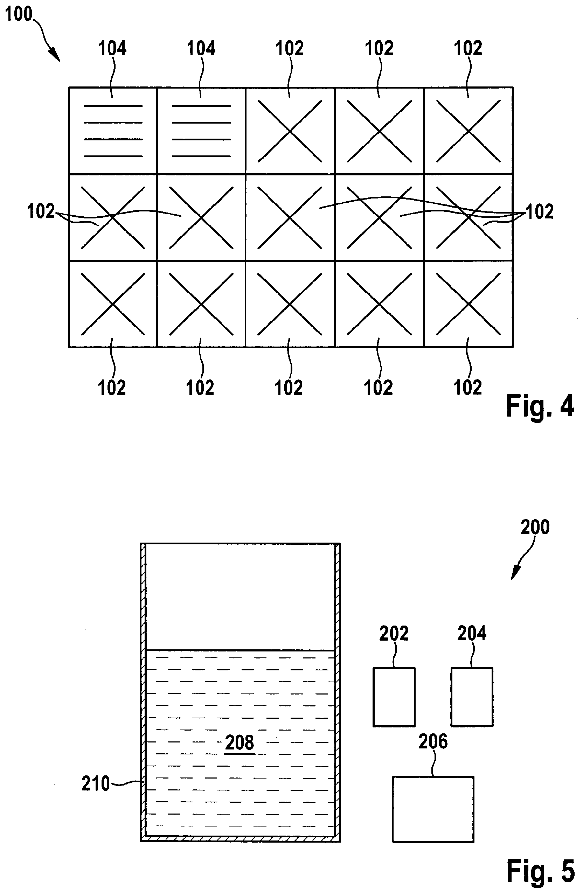

Figur 4 zeigt eine beispielhafte Anzeige 100 von Gärkurven, wie diese bspw. auf dem Smartphone und/oder der Basisstation gegeben werden kann. Die Anzeige umfasst eine Reihe von Feldern 102, die jeweils einem Behälter zugeordnet sein können. So hat der Nutzer eine Übersicht über eine Anzahl von Behältern. Über Bedienfelder 104 kann vorgegeben werden, welche Kurven, bspw. Zuckergehalt, Alkoholgehalt, Temperatur usw., in den Feldern 102 angezeigt werden. In den Feldern wird typischerweise die Behälternummer, der Gärstart, der Startzucker, der aktuelle Stand an Alkohol und Zucker angezeigt. Es können auch separate Felder vorgesehen sein, in denen nach Anwahl eines der Felder 102 diese und weitere Informationen zu dem zugeordneten Behälter angezeigt werden.

Figur 5 zeigt eine Ausführung einer Einrichtung, die insgesamt mit der Bezugsziffer 200 bezeichnet ist. Diese Einrichtung 200 umfasst ein Thermometer 202 und eine Anordnung zum Bestimmen eines Alkoholgehalts 204, in diesem Fall ein Refraktometer, und eine Basisstation 206. Die Einrichtung dient zum Überwachen der Gärung eines Gärgutes 208, das sich in einem Gärtank 210 befindet.

Mit der Anordnung 204 kann in regelmäßigen Abständen oder nach Bedarf die Zuckerabbaurate und gegebenenfalls der Alkoholgehalt des Gärgutes 208 bestimmt werden und somit ein Gärverlauf ermittelt werden. Das Thermometer 202 bestimmt nach Bedarf oder in regelmäßigen Abständen die Temperatur des Gärgutes 208. Bei dieser Ausführung werden die Alkoholwerte an das Thermometer 202 übermittelt, das wiederum die Werte zu Alkoholgehalt und Temperatur an die Basisstation, bspw. per Funk, übermittelt. In der Basisstation wird ermittelt, ob der Gärverlauf von einem gewünschten Verlauf abweicht und durch Beeinflussung der Temperatur auf diesen eingewirkt, d. h. es findet eine Regelung des Gärverlaufs bzw. der Gärung über die Temperatur statt.

**Patentansprüche**

1.   Verfahren zum Steuern einer Gärung eines Gärgutes (208), bei dem ein Gärverlauf überwacht und die Temperatur und die Zuckerabbaurate des Gärgutes (208) ermittelt werden, wobei durch Beeinflussung der Temperatur des Gärgutes (208) der Gärverlauf gesteuert wird, wobei eine Gärtemperaturregelung vorgenommen wird, **dadurch gekennzeichnet, dass** ein Temperaturkorrekturwert $T_2$ - $T_1$ aus der Gleichung

$$Q_{10} = \left(\frac{R_{Z-Soll}}{R_{Z-Ist}}\right)^{\frac{10K}{T_2-T_1}}$$

errechnet wird und daraus ein Wert für die Solltemperatur T2 ermittelt und eingestellt wird,
wobei $R_{Z-Ist}$ die momentane Zuckerabbaurate bei der ermittelten Temperatur (Isttemperatur) T1 bezeichnet und $R_{Z-Soll}$ die gewünschte Zuckerabbaurate bei der Solltemperatur T2 bezeichnet und Q10 der Faktor ist, um den die Zuckerabbaurate konkret steigt, wenn die Temperatur um 10K erhöht wird.

2.  Verfahren nach Anspruch 1, bei dem die Temperatur des Gärgutes (208) mit einem Thermometer (202) gemessen wird, das über eine Funkeinrichtung verfügt, so dass die Temperatur zu einer Basisstation (14, 206), bspw. ein PC oder ein Laptop, übermittelt werden kann, an der unter Berücksichtigung des Gärverlaufs die Beeinflussung der Temperatur zur Regelung des Gärverlaufs bewirkt wird.

3.  Verfahren zum Überwachen einer Gärung eines Gärgutes (208) nach Anspruch 1 oder 2, bei dem in zeitlichen Abständen mittels eines Refraktometers (12) ein Brechungsindex des Gärgutes (208) gemessen wird und auf Grundlage dieser Messung eine den aktuellen Zuckergehalt des Gärgutes bestimmende Messgröße bestimmt wird, wobei unter Berücksichtigung eines Anfangswerts für den Zuckergehalt aus dem aktuellen Zuckergehalt ein aktueller Alkoholgehalt bestimmt wird und auf diese Weise eine Gärkurve erstellt wird.

4.  Verfahren nach Anspruch 3, bei dem als Anfangswert für die den aktuellen Zuckergehalt des Gärgutes (208) bestimmende Messgröße des Gärgutes (208) in unvergorenem Zustand herangezogen wird, der durch Messung vor Beginn der Gärung ermittelt wird.

5.  Verfahren nach Anspruch 3 oder 4, bei dem der aktuelle Zuckergehalt mit folgender Formel berechnet wird:

$$ABK = AB * K0\_Fak + SB * (1 - K0\_Fak),$$

    wobei SB der Anfangs-Zuckerwert, AB der aktuelle Zuckerwert, ABK der korrigierte aktuelle Zuckerwert und K0_Fak ein Umrechnungsfaktor ist.

6.  Verfahren nach Anspruch 5, bei dem der Umrechnungsfaktor als nicht konstant angenommen wird.

7.  Verfahren nach Anspruch 6, bei dem der Umrechnungsfaktor mit fortlaufender Gärung zunimmt.

8.  Verfahren nach einem der Ansprüche 3 bis 7, bei dem vom Refraktometer ermittelte Ergebnisse an eine Basisstation (14, 206), bspw. ein PC oder ein Laptop, übermittelt werden.

9.  Verfahren nach einem der Ansprüche 1 bis 8, bei dem zur Übermittlung von Ergebnissen ein Smartphone eingesetzt wird.

10. Verfahren nach einem der Ansprüche 1 bis 9, bei dem der Faktor Q10 ein Wert zwischen 2 und 4 angesetzt wird.

11. Einrichtung zum Steuern einer Gärung eines Gärgutes (208), zur Durchführung eines Verfahrens nach einem der Ansprüche 1 bis 10, mit einer Steuereinrichtung, die dazu eingerichtet ist, einen Gärverlauf des Gärgutes (208) durch Beeinflussung der Temperatur des Gärgutes (208) zu steuern, **dadurch gekennzeichnet, dass** die Einrichtung dazu eingerichtet ist, eine Gärtemperaturregelung vorzunehmen, einen Temperaturkorrekturwert $T_2 - T_1$ aus der Gleichung

$$Q_{10} = \left(\frac{R_{Z-Soll}}{R_{Z-Ist}}\right)^{\frac{10K}{T_2 - T_1}}$$

    zu errechnen und daraus einen Wert für die Solltemperatur $T_2$ zu ermitteln und einzustellen,
    wobei $R_{Z-Ist}$ die momentane Zuckerabbaurate bei der ermittelten Temperatur (Isttemperatur) $T1$ bezeichnet und $R_{Z-Soll}$ die gewünschte Zuckerabbaurate bei der Solltemperatur $T2$ bezeichnet und $Q10$ der Faktor ist, um den die Zuckerabbaurate konkret steigt, wenn die Temperatur um 10K erhöht wird.

12. Computerprogramm mit Programmcodemitteln, um ein Verfahren nach einem der Ansprüche 1 bis 10 durchzuführen, wenn das Computerprogramm auf einem Mikroprozessor eines Computers ausgeführt wird.

13. Computerprogrammprodukt mit Programmcodemitteln, die auf einem computerlesbaren Datenträger gespeichert sind, um ein Verfahren nach einem der Ansprüche 1 bis 10 durchzuführen, wenn das Computerprogramm auf einem Mikroprozessor eines Computers ausgeführt wird.

die dazu ausgebildet ist, in zeitlichen Abständen einen Brechungsindex des Gärgutes zu messen und auf Grundlage dieser Messung einen aktuellen Zuckergehalt des Gärgutes zu bestimmen, unter Berücksichtigung eines Anfangswerts für den Zuckergehalt aus dem aktuellen Zuckergehalt einen aktuellen Alkoholgehalt zu bestimmen und auf diese Weise eine Gärkurve zu erstellen.

12. Messeinrichtung nach Anspruch 11, bei der das Refraktometer und die Basisstation jeweils über eine Schnittstelle für eine drahtlose Kommunikation verfügen.

13. Messeinrichtung nach Anspruch 11, die ein Smartphone zur Übermittlung von Ergebnissen umfasst.

14. Computerprogramm mit Programmcodemitteln, um ein Verfahren nach einem der Ansprüche 1 bis 9 durchzuführen, wenn das Computerprogramm auf einem Mikroprozessor eines Computers, insbesondere in einer Messeinrichtung nach einem der Ansprüche 11 bis 13, ausgeführt wird.

15. Computerprogrammprodukt mit Programmcodemitteln, die auf einem computerlesbaren Datenträger gespeichert sind, um ein Verfahren nach einem der Ansprüche 1 bis 9 durchzuführen, wenn das Computerprogramm auf einem Mikroprozessor eines Computers, insbesondere in einer Messeinrichtung nach einem der Ansprüche 11 bis 13, ausgeführt wird.

## Claims

1. A method for controlling fermentation of a fermenting substance (208), in which a fermentation process is monitored and the temperature and sugar breakdown rate of the fermenting substance (208) are determined, wherein the fermentation process is controlled by influencing the temperature of the fermenting substance (208), wherein a fermentation temperature regulation is performed, **characterized in that** a temperature correction value $T_2 - T_1$ from the equation

$$Q_{10} = \left(\frac{R_{Z-Soll}}{R_{Z-Ist}}\right)^{\frac{10K}{T_2-T_1}}$$

is calculated and from this, a value for the target temperature T2 is determined and set, wherein $R_{Z-Ist}$ is the current sugar breakdown rate at the determined temperature (actual temperature) T1, and $R_{Z-Soll}$ is the desired sugar breakdown rate at the target temperature T2, and Q10 is the factor by which the sugar breakdown rate concretely rises when the temperature is increased by 10 K.

2. The method according to claim 1 in which the temperature of the fermenting substance (208) is measured with a thermometer (202) that has a radio device, such that the temperature can be transmitted to a base station (14, 206), e.g., a PC or a laptop, by which the temperature is influenced to regulate the fermentation process, taking into account the fermentation process.

3. The method for monitoring a fermentation of a fermenting substance (208) according to claims 1 or 2 in which an index of refraction of the fermenting substance (208) is measured at time intervals by means of a refractometer (12) and upon the basis of this measurement, a measured value determining the current sugar content of the fermenting substance is determined, wherein a current alcohol content is determined from the current sugar content taking into account an initial value for the sugar content, and in this manner, a fermentation curve is produced.

4. The method according to claim 3 in which the measured value of the fermenting substance (208) in the unfermented state determined by measurement before fermentation begins is to be used as the initial value for the current sugar content of the fermenting substance (208).

5. The method according to claim 3 or 4 in which the current sugar content is calculated using the following formula:

$$ABK = AB * K0\_Fak + SB * \quad (1 - K0\_Fak),$$

where SB is the initial sugar value, AB is the current sugar value, ABK is the corrected current sugar value and K0_Fak is a conversion factor.

6. The method according to claim 5 in which the conversion factor is presumed to be non-constant.

7.  The method according to claim 6 in which the conversion factor increases as fermentation progresses.

8.  The method according to one of claims 3 to 7 in which results determined by the refractometer are conveyed to a base station (14, 206), e.g., a PC or a laptop.

9.  The method according to one of claims 1 to 8 in which a smartphone is used to convey results.

10. The method according to one of claims 1 to 9 in which a value between 2 and 4 is used for the factor Q10.

11. An apparatus for controlling a fermentation of a fermenting substance (208), for executing a method according to one of claims 1 to 10, with a control unit configured to control a fermentation process of the fermenting substance (208) by influencing the temperature of the fermenting substance (208), **characterized in that** the apparatus is configured to perform a fermentation temperature regulation, to calculate a temperature correction value $T_2 - T_1$ from the equation

$$Q_{10} = \left(\frac{R_{Z-Soll}}{R_{Z-Ist}}\right)^{\frac{10K}{T_2 - T_1}}$$

and based on this, to determine and set a value for the target temperature $T_2$, where $R_{Z-Ist}$ is the current sugar breakdown rate at the determined temperature (actual temperature) T1 and $R_{Z-Soll}$ is the desired sugar breakdown rate at the target temperature T2 and Q10 is the factor by which the sugar breakdown rate concretely rises when the temperature is increased by 10 K.

12. A computer program with program code means to execute a method according to one of claims 1 to 10 when the computer program is executed on a microprocessor of a computer.

13. A computer program product with program code means saved on a computer-readable data carrier for executing a method according to one of claims 1 to 10 when the computer program is executed on a microprocessor of a computer.

**Revendications**

1.  Procédé de contrôle de la fermentation d'une substance en fermentation (208), selon lequel un processus de fermentation est surveillé et la température et le taux de dégradation du sucre de la substance en fermentation (208) sont déterminés, le processus de fermentation étant contrôlé par une action sur la température de la substance en fermentation (208), une régulation de la température de fermentation étant entreprise, **caractérisé en ce qu'**une valeur de correction de température $T_2 - T_1$ est calculée à partir de l'équation

$$Q_{10} = \left(\frac{R_{Z-Soll}}{R_{Z-Ist}}\right)^{\frac{10K}{T_2 - T_1}}$$ ,

valeur à partir de laquelle une valeur pour la température de consigne T2 est déterminée et réglée, $R_{Z-Ist}$ désignant le taux instantané de dégradation du sucre à la température déterminée (température réelle) T1, et $R_{Z-Soll}$ désignant le taux souhaité de dégradation du sucre à la température de consigne T2, et Q10 étant le facteur dont le taux de dégradation du sucre augmente concrètement lorsque la température est augmentée de 10K.

2.  Procédé selon la revendication 1, selon lequel la température de la substance en fermentation (208) est mesurée avec un thermomètre (202) qui dispose d'un équipement radio, de sorte que la température peut être communiquée à une station de base (14, 206), par exemple un ordinateur de bureau ou un ordinateur portable où est produite, en tenant compte du processus de fermentation, l'action sur la température afin de réguler le processus de fermentation.

3.  Procédé de surveillance de la fermentation d'une substance en fermentation (208) selon la revendication 1 ou 2, selon lequel un indice de réfraction de la substance en fermentation (208) est mesuré à intervalles de temps au moyen d'un réfractomètre (12) et, sur la base de ladite mesure, un paramètre définissant la teneur actuelle en sucre de la substance en fermentation est déterminé, sachant qu'en tenant compte d'une valeur initiale pour la teneur en sucre, une teneur actuelle en alcool est déterminée à partir de la teneur actuelle en sucre et une courbe de fermentation est ainsi établie.

4. Procédé selon la revendication 3, selon lequel on utilise, comme valeur initiale pour le paramètre de la substance en fermentation (208) définissant la teneur actuelle en sucre de la substance en fermentation (208), la teneur en sucre de la substance à l'état non fermenté, qui est déterminée en la mesurant avant le début de la fermentation.

5. Procédé selon la revendication 3 ou 4, selon lequel la teneur actuelle en sucre est calculée avec la formule suivante :

$$ABK = AB * K0\_Fak + SB * \quad (1 - K0\_Fak),$$

SB étant la valeur de sucre initiale, AB la valeur de sucre actuelle, ABK la valeur de sucre actuelle corrigée et K0_Fak un facteur de conversion.

6. Procédé selon la revendication 5, selon lequel le facteur de conversion est supposé ne pas être constant.

7. Procédé selon la revendication 6, selon lequel le facteur de conversion augmente avec la progression de la fermentation.

8. Procédé selon l'une quelconque des revendications 3 à 7, selon lequel les résultats déterminés par le réfractomètre sont communiqués à une station de base (14, 206), par exemple un ordinateur de bureau ou un ordinateur portable.

9. Procédé selon l'une quelconque des revendications 1 à 8, selon lequel on utilise un smartphone pour la communication des résultats.

10. Procédé selon l'une quelconque des revendications 1 à 9, selon lequel on applique au facteur Q10 une valeur comprise entre 2 et 4.

11. Dispositif pour le contrôle de la fermentation d'une substance en fermentation (208), pour la mise en oeuvre d'un procédé selon l'une quelconque des revendications 1 à 10, avec un dispositif de contrôle qui est conçu pour contrôler un processus de fermentation de la substance en fermentation (208) par une action sur la température de la substance en fermentation (208), **caractérisé en ce que** le dispositif est conçu pour entreprendre une régulation de la température de fermentation, pour calculer une valeur de correction de température $T_2$ - $T_1$ à partir de l'équation

$$Q_{10} = \left(\frac{R_{Z-Soll}}{R_{Z-Ist}}\right)^{\frac{10K}{T_2-T_1}},$$

et pour déterminer et régler à partir de ladite valeur une valeur pour la température de consigne T2, $R_{Z-Ist}$ désignant le taux instantané de dégradation du sucre à la température déterminée (température réelle) T1, et $R_{Z-Soll}$ désignant le taux souhaité de dégradation du sucre à la température de consigne T2, et Q10 étant le facteur dont le taux de dégradation du sucre augmente concrètement lorsque la température est augmentée de 10K.

12. Programme informatique avec des moyens de code de programme, afin de mettre en oeuvre un procédé selon l'une quelconque des revendications 1 à 10 lorsque le programme informatique est exécuté sur un microprocesseur d'un ordinateur.

13. Produit-programme informatique avec des moyens de code de programme qui sont enregistrés sur un support de données pouvant être lu par ordinateur, afin de mettre en oeuvre un procédé selon l'une quelconque des revendications 1 à 10 lorsque le programme informatique est exécuté sur un microprocesseur d'un ordinateur.

Fig. 1

Fig. 2

Fig. 3

100

104    104    102    102    102

102                                            102

102    102    102    102    102

**Fig. 4**

200

202    204

206

208

210

**Fig. 5**

**IN DER BESCHREIBUNG AUFGEFÜHRTE DOKUMENTE**

**In der Beschreibung aufgeführte Patentdokumente**

- DE 3787515 T2
- CN 201955301 U
- EP 1559775 A2


**In der Beschreibung aufgeführte Nicht-Patentliteratur**

- **GEORGE COJOCARU et al.** *A new refractometric methodology used to monitor fermentations*
- Muti-purpose optoelectronic instrument for monitoring the alcoholic fermentation of wine. F. Jimenez et a Sensoring Proceedingsl. IEEE, 2011